# EUROPEAN PATENT SPECIFICATION

(11) **EP 1 466 596 B1**
(45) Date of publication and mention of the grant of the patent: **31.08.2016**
(21) Application number: 02790851.6
(22) Date of filing: 25.12.2002
(51) Int. Cl.: A61K 9/16, B01J 13/12, A61K 47/26

(54) **MICROSPHERE AND METHOD FOR PRODUCTION THEREOF**
MIKROSPHÄRE UND VERFAHREN ZU DEREN HERSTELLUNG
MICROSPHERE ET SON PROCÉDÉ DE PRODUCTION

(30) Priority: 26.12.2001 JP 2001394663
(43) Date of publication of application: 13.10.2004
(73) Proprietor: Takeda Pharmaceutical Company Limited, Osaka (JP)
(72) Inventor: FUTO, Tomomichi, Ikeda-shi, Osaka 563-0033 (JP); YAMAMOTO, Kazumichi, Nakagyo-ku Kyoto-shi, Kyoto 604-0911 (JP); ARAI, Jiichi, Kobe-shi, Hyogo 658-0015 (JP)
(74) Representative: Kingsbury, Oliver William
(86) International application number: PCT/JP2002/013476
(87) International publication number: WO 2003/055470

(56) References cited:
- EP-A- 0 582 459
- EP-A- 0 633 020
- EP-A- 1 048 301
- EP-A- 1 532 985
- EP-A1- 0 481 732
- WO-A1-98/32423
- JP-A- 10 203 962
- JP-A- 2002 020 269

## Description

### Technical Field

The present invention relates to the use of mannitol to produce a microsphere having improved dispersibility.

### Background Art

For example, Japanese Patent Application Laid-Open (JP-A) Nos. 57-118512, 57-150609 and 6-145046 disclose a method of producing a sustained-release microsphere from a W/O type emulsion using a biodegradable polymer.

The sustained-release biodegradable polymer is useful as, for example, a base material for a physiologically active substance-enclosing microsphere or the like. Known examples of such a biodegradable polymer include polylactic acid and a copolymer of lactic acid and glycolic acid (e.g., JP-A 11-269094).

After produced by any conventional synthesis method, such biodegradable polymers have been used as they are. It has been found, however, that such unmodified product as synthesized has a low amount of the terminal carboxyl group and thus can be less useful as a sustained-release base material. Thus, investigations have been made on a process including the steps of hydrolyzing such unmodified biodegradable polymer with a high molecular weight to form a product having an appropriate weight average molecular weight and then using the product as a base material for a sustained-release preparation.

However, the product obtained after the hydrolysis and washing with water can easily cause an initial burst and thus is not suitable as the sustained-release base material, even though it has an appropriate weight average molecular weight and an appropriate amount of the terminal carboxyl group. Thus, there has been a demand for its improvement under the present circumstances.

JP-A 7-97334 discloses a sustained-release preparation and a method of producing the same, wherein the preparation comprises a physiologically active peptide or a salt thereof and a biodegradable polymer having a free carboxyl group at its terminal.

EP0582459 relates to a process for producing microcapsules of a water-soluble drug by an in-water drying process, in which an O/W emulsion is added to an external aqueous phase comprising an osmotic pressure adjustor.

EP1048301 relates to a sustained-release composition containing a hydroxynaphthoic salt of a biologically active substance and a biodegradable polymer, which can be produced using an external water phase having an osmotic pressure adjustor.

EP0633020 relates to a method of producing a sustained-release preparation, which involves the permeation of a water-soluble polypeptide into a biodegradeable matrix in an aqueous solution.

EP0481732 relates to a polymer for use in a prolonged-release preparation, and describes a method for incorporating a physiologically active peptide by mixing a water oil emulsion containing the polymer and peptide with an external aqueous phase.

WO98/32423discloses the production of microcapsules of leuprorelin acetate by subjecting a W/O emulsion to an in-water drying process, which is carried out in the presence of mannitol in the external phase.

However, these literatures are silent on any method for improving the dispersibility of the microsphere.

It is therefore an object of the present invention to provide a microsphere having improved dispersibility, a method of producing the same, and so on.

### Disclosure of Invention

The present inventors have made active investigations with the above object in view and consequently found that in an in-water drying process for production of microspheres, addition of an osmotic pressure regulating agent to the outer aqueous phase can surprisingly improve the dispersibility of the microsphere product. Based on the finding, the present inventors have further made investigations and finally completed the present invention.

Thus, the present invention provides:
(1) The use of mannitol for improving dispersibility of microspheres, in which the mannitol is added in an outer aqueous phase in producing microspheres by an in-water drying method using a W/O type emulsion, wherein the W/O type emulsion consists of: 1) an inner aqueous phase containing a peptide represented by the formula: 5-oxo-Pro-His-Trp-Ser-Tyr-Y-Leu-Arg-Pro-Z wherein Y represents DLeu, DAla, DTrp, DSer(tBu), D2Na1 or DHis(ImBzl) and Z represents NH-C₂H₅ or Gly-NH₂ or a salt thereof and
2) an oil phase of a solution containing a lactic acid polymer with a weight average molecular weight of 15000 to 50000 or a salt thereof; and subjecting the dispersion to an in-water drying method.

### Brief Description of Drawings

Fig. 1 is an electron micrograph showing microspheres of Comparative Example 1.
Fig. 2 is an electron micrograph showing microspheres of Example 1.

### Best Mode for Carrying Out the Invention

The physiologically active substance for use in the present invention has high hydrophilicity and low n-octanol/water (oil/water) partition ratio. Such low oil/water partition ratio means an n-octanol/water solubility ratio of preferably 1 or less, more preferably 0.1 or less.

The oil/water partition ratio can be determined by the method described in Jitsusaburo Samejima, "Butsuri Kagaku Jikkenho (Physicochemical Experimental Method)", published by SHOKABO PUBLISHING Co., Ltd., 1961. Specifically, the method is performed as follows. First, n-octanol and a buffer, pH 5.5 (a 1:1 mixture) are put in a test tube. For example, the buffer is Sφerensen buffer [Ergeb. Physiol., 12, 393 (1912)], Clark-Lubs buffer [J. Bact., 2(1), 109, 191 (1917)], Macllvaine buffer [J. Biol. Chem., 49, 183 (1921)], Michaelis buffer [Die Wasser-stoffionenkonzentration, p. 186 (1914)], Kolthoff buffer [Biochem. Z., 179, 410 (1926)], or the like. An appropriate amount of a drug is put in the test tube. The test tube is then capped and immersed in a thermostatic bath (25°C) while vigorously shaken frequently. When the drug appears to be dissolved in both liquid layers to reach equilibrium, the liquid mixture is allowed to stand or be centrifuged. A certain amount of the liquid is then pipetted from each of the upper and lower layers, and analyzed so that the concentration of the drug in each layer is determined. The oil/water partition ratio is obtained as the ratio of the concentration of the drug in the n-octanol layer to that in the water layer.

The physiologically active substance includes physiologically active peptides represented by the general formula [II]:
5-oxo-Pro-His-Trp-Ser-Tyr-Y-Leu-Arg-Pro-Z
wherein Y represents a residue selected from DLeu, DAla, DTrp, DSer(tBu), D2Nal and DHis(ImBzl), and Z represents NH-C₂H₅ or Gly-NH₂, and salts thereof. Particularly preferred is a peptide wherein Y is DLeu and Z is NH-C₂H₅ (that is, peptide A represented by the formula: 5-oxo-Pro-His-Trp-Ser-Tyr-DLeu-Leu-Arg-Pro-NH-C₂H₅, leuprorelin) or a salt (such as an acetate) thereof.

These peptides can be produced by or based on the method as disclosed in "Treatment with GnRH analogs:
Controversies and perspectives" published by The Parthenon Publishing Group Ltd., 1996, JP-A 3-503165, JP-A 3-101695, JP-A 7-97334 and JP-A 8-259460.

The abbreviations used herein have the following meanings, respectively:

| Abbreviations: | Names |
|---|---|
| N(4H₂-furoyl)Gly: | N-tetrahydrofuroylglycine residue |
| NAc: | N-acetyl group |
| D2Nal: | D-3-(2-naphthyl)alanine residue |
| D4ClPhe: | D-3-(4-chloro)phenylalanine residue |
| D3Pal: | D-3-(3-pyridyl)alanine residue |
| NMeTyr: | N-methylthyrosine residue |
| Aph(Atz): | N-[5'-(3'-amino-1'H-1',2',4'-triazolyl)]phenylalanine residue |
| NMeAph(Atz): | N-methyl-[5'-(3'-amino-1'H-1',2',4'-triazolyl)]phenylalanine residue |
| DLys (Nic) : | D-(e-N-nicotinoyl)lysine residue |
| Dcit: | D-citrulline residue |
| DLys(AzaglyNic): | D-(azaglycylnicotinoyl)lysine residue |
| DLys(AzaglyFur): | D-(azaglycylfuranyl)lysine residue |
| DhArg(Et₂): | D-(N,N'-diethyl)homoarginine residue |
| DAph(Atz): | D-N-[5'-(3'-amino-1'H-1',2',4'-triazolyl)]phenylalanine residue |
| DhCi: | D-homocitrulline residue |
| Lys (Nisp) : | (e-N-isopropyl)lysine residue |
| hArg(Et₂): | (N,N'-diethyl)homoarginine residue |

Abbreviations for other amino acids are according to those defined by the IUPAC-IUB Commission on Biochemical Nomenclature or defined in European Journal of Biochemistry Vol. 138, pp. 9-37, 1984 or according to conventional abbreviations in the field. If not stated otherwise, amino acids are in L-configuration, although they may have optical isomers.

If the physiologically active substance has a basic group such as an amino group, such a salt may be a salt of an inorganic acid (or a free inorganic acid) (such as carbonic acid, bicarbonic acid, hydrochloric acid, sulfuric acid, nitric acid, and boric acid) or a salt of an organic acid (or a free organic acid) (such as succinic acid, acetic acid, propionic acid, and trifluoroacetic acid).

If the physiologically active substance has an acidic group such as a carboxyl group, such a salt may be a salt of an inorganic base (or a free inorganic base) (such as an alkali metal such as sodium and potassium and an alkaline earth metal such as calcium and magnesium) or a salt of an organic base (or a free organic base) (such as an organic amine such as triethylamine and a basic amino acid such as arginine). The physiologically active peptide may also form a metal complex compound (such as a copper complex and a zinc complex).

The polymer for use in the present invention is less soluble or insoluble in water and biocompatible (biodegradable). The wording "less soluble in water" means that the polymer has a solubility of not less than 0 and not more than about 3% (w/w) in water, preferably a solubility of not less than 0 and not more than about 1% (w/w) in water.

The biodegradable polymer to be used may have a weight average molecular weight of about 15000 to 50000, more preferably of about 15000 to 40000, particularly preferably of about 17000 to 26000. The biodegradable polymer may have a dispersibility of about 1.2 to 4.0, particularly preferably of about 1.5 to 3.5.

As used herein, the weight average molecular weight and the dispersibility each means a value determined by Gel Permeation Chromatography (GPC).

The amount of the polymer to be used depends on the pharmacological activity and the rate and period of release of the physiologically active substance or a salt thereof and the like. For example, the polymer may be used as a microsphere base material in an amount of about 0.5 to 10,000 times (by weight), preferably of about 1 to 100 times (by weight) the amount of the physiologically active substance or a salt thereof.

The polymer is a lactic acid polymer (hereinafter, such a lactic acid polymer is also simply referred to as the lactic acid polymer of the present invention) consisting of only lactic acid. In such a polymer, the content of a polymer with a weight average molecular weight of 5000 or less is generally about 10% by weight or less, preferably, the content of a polymer with a weight average molecular weight of 5000 or less is generally about 5% by weight or less; more preferably, the content of a polymer with a weight average molecular weight of 3000 or less is about 1.5% by weight or less; still preferably, the content of a polymer with a weight average molecular weight of 1000 or less is about 0.1% by weight or less; still more preferably, the content of a polymer with a weight average molecular weight of 5000 or less is about 5% by weight or less, and the content of a polymer with a weight average molecular weight of 3000 or less is about 1.5% by weight or less; most preferably, the content of a polymer with a weight average molecular weight of 5000 or less is about 5% by weight or less, the content of a polymer with a weight average molecular weight of 3000 or less is about 1.5% by weight or less, and the content of a polymer with a weight average molecular weight of 1000 or less is about 0.1% by weight or less.

The lactic acid polymer of the present invention generally has a weight average molecular weight of 15000 to 50000, preferably of 15000 to 40000, more preferably of 17000 to 26000, particularly preferably of 17500 to 25500.

A high molecular weight lactic acid polymer for use as material for the lactic acid polymer of the present invention may be commercially available or may be produced by any known polymerization method and generally has a weight average molecular weight of 15000 to 500000. Examples of the known polymerization method include condensation polymerization of lactic acid, ring-opening polymerization of, for example, lactide with a Lewis acid such as diethyl zinc, triethylaluminum and tin octylate or a catalyst such as a metal salt, ring-opening polymerization of lactide in the presence of a hydroxycarboxylic acid derivative with the protected carboxyl group, in addition to the conditions of the above mentioned ring-opening polymerization (for example, International Patent Publication WO00/35990), ring-opening polymerization of lactide with a catalyst under heating (for example, J. Med. Chem., 16, 897 (1973)).

The type of the polymerization may be bulk polymerization in which lactide or the like is melted and subjected to polymerization reaction or solution polymerization in which lactide or the like is dissolved in a suitable solvent and subjected to polymerization reaction. A polymer produced by the solution polymerization is industrially preferred for use as material for the lactic acid polymer of the present invention.

Examples of a solvent for dissolving lactide during the solution polymerization include aromatic hydrocarbons such as benzene, toluene and xylene, decalin, and dimethylformamide.

The resulting lactic acid polymer with a high molecular weight may be hydrolyzed by any known hydrolysis method. For example, the lactic acid polymer with the high molecular weight is dissolved in a suitable solvent and then water and optionally an acid are added thereto to lead to hydrolysis.

The solvent for dissolving the high molecular weight lactic acid polymer may be any solvent, as long as the lactic acid polymer can be dissolved in such a solvent in an amount of not more than 10 times (by weight) the amount of the polymer. Specific examples of such a solvent include a halogenated hydrocarbon such as chloroform and dichloromethane; an aromatic hydrocarbon such as toluene, o-xylene, m-xylene and p-xylene; cyclic ether such as tetrahydrofuran; acetone; and N,N-dimethylformamide. If the solvent used in the preparation of the high molecular weight lactic acid polymer is also applicable to the hydrolysis of the polymer, the polymerization and the hydrolysis may be continuously performed without isolation of the high molecular weight lactic acid polymer.

The amount of the solvent to be used for dissolving the high molecular weight lactic acid polymer is generally 0.1 to 100 times, preferably 1 to 10 times the amount of the lactic acid polymer to be a solute.

The amount of water to be added is generally 0.001 to 1 time (by weight), preferably of 0.01 to 0.1 times (by weight) the amount of the high molecular weight lactic acid polymer.

Examples of the acid to be optionally added include inorganic acid such as hydrochloric acid, sulfuric acid and nitric acid; and organic acid such as lactic acid, acetic acid and trifluoroacetic acid, and lactic acid is preferred.

The amount of such an acid to be added is generally 0 to 10 times (by weight), preferably of 0.1 to 1 time (by weight) the amount of the high molecular weight lactic acid polymer.

The reaction temperature of the hydrolysis is generally from 0 to 150°C, preferably from 20 to 80°C.

The reaction time of the hydrolysis may vary depending on the weight average molecular weight of the high molecular weight lactic acid polymer and the reaction temperature, and is generally from 10 minutes to 100 hours, preferably from 1 to 20 hours.

The timing of stopping the hydrolysis process may be determined based on the weight average molecular weight of the hydrolysis product. Specifically, samples are taken at any appropriate time during the hydrolysis process, the weight average molecular weight of the hydrolysis product in the sample is measured by Gel Permeation Chromatography (GPC), and then the hydrolysis process is stopped if the molecular weight is determined as being from about 15000 to 50000, preferably from about 15000 to 30000, more preferably from about 17000 to 26000, particularly preferably from 17500 to 25500.

After the above described hydrolysis of the high molecular weight lactic acid polymer, the desired lactic acid polymer may be precipitated from the resulting solution which contains the hydrolysis product. For example, the hydrolysis product-containing solution is brought into contact with a solvent that can induce precipitation of the desired lactic acid polymer.

In a preferred aspect, the hydrolysis product-containing solution is a 10 to 50 wt% solution of the lactic acid polymer with a weight average molecular weight of 15000 to 50000, preferably of 15000 to 30000, more preferably of 17000 to 26000, particularly preferably of 17500 to 25500 in a solvent capable of dissolving a high molecular weight lactic acid polymer, for example, halogenated hydrocarbon such as chloroform and dichloromethane; aromatic hydrocarbon such as toluene, o-xylene, m-xylene and p-xylene; cyclic ether such as tetrahydrofuran; acetone; N,N-dimethylformamide; dichloromethane; or xylene.

Examples of a solvent with which the desired lactic acid polymer can be precipitated from the hydrolysis product-containing solution include alcohols such as methanol and ethanol, chain ethers such as isopropyl ether, aliphatic hydrocarbons such as hexane, and water.

The amount of the solvent to be used in order to precipitate the desired lactic acid polymer is generally 0.1 to 100 times (by weight), preferably of 1 to 10 times (by weight) the amount of a solvent of the hydrolysis product-containing solution.

Preferred combination of the type and amount of each solvent may be a combination of a hydrolysis product-containing solution in which dichloromethane in an amount of 1 to 5 times (by weight) the amount of the solute is used and isopropyl ether as a solvent for reducing the solubility in an amount of 2 to 10 times (by weight) the amount of the dichloromethane.

When the hydrolysis product-containing solution is contacted with the solvent for precipitating the desired lactic acid polymer, the temperature of the solvent is generally set at -20 to 60°C, preferably 0 to 40°C, and the temperature of the hydrolysis product-containing solution is generally set at 0 to 40°C, preferably 10 to 30°C.

Examples of a method for contacting the solvent with the hydrolysis product-containing solution include a method of adding the hydrolysis product-containing solution to the solvent at a time, a method of adding the hydrolysis product-containing solution dropwise to the solvent, a method of adding the solvent to the hydrolysis product-containing solution at a time, and a method of adding the solvent dropwise to the hydrolysis product-containing solution.

The lactic acid polymer of the present invention obtained as shown above is preferably used as a base material for a sustained-release preparation, because the amount of its terminal carboxyl group is in a preferred range for such a base material.

The concentration of the polymer in an oil phase may be from about 0.5 to about 90% (w/w), preferably from about 2 to about 60% (w/w).

Examples of the drug carrier to be used in the present invention include albumin, gelatin, citric acid, salicylic acid, sodium ethylenediaminetetraacetate, dextrin, sodium hydrogen sulfite, polyol compounds such as polyethylene glycol, agar, alginic acid, polyvinyl alcohol, and a basic amino acid.

The microsphere of the present invention may be produced by an in-water drying method, preferably by a (W/O)/W type, O/W type or S/O/W type in-water drying method.

The (W/O)/W type in-water drying method may comprise preparing a W/O type emulsion that consists of an inner aqueous phase of a liquid containing a physiologically active substance or a salt thereof and an oil phase of a solution containing a polymer; dispersing the emulsion into an aqueous phase containing an osmotic pressure regulating agent to prepare a (W/O)/W type emulsion; and subjecting the emulsion to an in-water drying method to remove a solvent from the oil phase so that microspheres are produced which contain the physiologically active substance or a salt thereof and the polymer.

The O/W type in-water drying method may comprise dispersing an oil phase into an aqueous phase containing an osmotic pressure regulating agent to prepare an O/W type emulsion, wherein the oil phase comprises a physiologically active substance or a salt thereof and a polymer; and subjecting the emulsion to an in-water drying method to remove a solvent from the oil phase so that microspheres are produced which contain the physiologically active substance or a salt thereof and the polymer.

The S/O/W type in-water drying method may comprise dispersing a physiologically active substance or a salt thereof into an oil phase of a solution containing a polymer; dispersing the dispersion into an aqueous phase containing an osmotic pressure regulating agent to prepare an S/O/W type emulsion; and subjecting the emulsion to an in-water drying method to remove a solvent from the oil phase so that microspheres are produced which contain the physiologically active substance or a salt thereof and the polymer.

The osmotic pressure regulating agent to be used in the present invention may be mannitol.

The amount of the physiologically active substance or a salt thereof to be used may vary depending on the type of the drug, the desired pharmacological effect, the desired effect duration, or the like. For example, the concentration of the physiologically active substance or a salt thereof in an inner aqueous phase is from about 0.001% to about 90% (w/w), more preferably from about 0.01% to about 80% (w/w), particularly preferably from about 0.01% to about 70% (w/w).

The osmotic pressure regulating agent may be used at such a concentration that an outer aqueous phase has an osmotic pressure of about 1/50 to about 5 times, preferably of about 1/25 to about 3 times, more preferably of about 1/12 to about 2 times the osmotic pressure of isotonic sodium chloride solution.

Specifically, if the osmotic pressure regulating agent is a nonionic substance, the concentration in an outer aqueous phase is from about 0.01% to about 60% (w/w), preferably from about 0.01% to about 40% (w/w), more preferably from about 0.05% to about 30% (w/w), particularly preferably from about 0.5% to about 1.5% (w/w). If the osmotic pressure regulating agent is an ionic substance, the concentration may be calculated by dividing the above concentration by its total ionic valence. The concentration of the added osmotic pressure regulating agent does not have to be equal to or lower than its solubility, and it may be partially in a dispersed state.

According to the present invention, the addition of the osmotic pressure regulating agent to an outer aqueous phase can provide improved dispersibility of the microsphere product. The degree of such improvement is not particularly limited, but preferred is, for example, such a degree that about 400 to 700 mg of the microspheres can be dispersed in 1.5 ml of a dispersion medium for injection in less than 2 minutes.

Hereinafter, a method of producing microspheres by a (W/O)/W type-in-water drying method according to the present invention is explained.

In the process as described below, the following ingredients may be added to an inner aqueous phase as needed:
(1) Drug carrier: albumin, gelatin, citric acid, salicylic acid, sodium ethylenediaminetetraacetate, dextrin, sodium hydrogen sulfite, polyol compounds such as polyethylene glycol, agar, alginic acid, polyvinyl alcohol, basic amino acid, or the like;
(2) pH regulator for keeping the stability and solubility of a physiologically active substance or a salt thereof: carbonic acid, acetic acid, oxalic acid, citric acid, phosphoric acid, hydrochloric acid, sodium hydroxide, arginine, lysine, a salt thereof, or the like;
(3) Stabilizer for a physiologically active substance or the salt thereof: albumin, gelatin, citric acid, sodium ethylenediaminetetraacetate, dextrin, sodium hydrogen sulfite, polyol compounds such as polyethylene glycol, or the like;
(4) Preservative: para-hydroxybenzoate esters (such as methyl paraben and propyl paraben), benzyl alcohol, chlorobutanol, thimerosal, or the like.

### (I) O/W Method

For this method, first, a solution of the polymer in an organic solvent is prepared. The organic solvent for use in production of the microspheres of the present invention preferably has a boiling point of 120°C or lower.

Examples of such an organic solvent include halogenated hydrocarbon (such as dichloromethane, chloroform, dichloroethane, trichloroethane, and carbon tetrachloride), ether (such as ethyl ether and isopropyl ether), fatty acid ester (such as ethyl acetate and butyl acetate), aromatic hydrocarbon (such as benzene, toluene and xylene), alcohol (such as ethanol and methanol), and acetonitrile. Preferred is halogenated hydrocarbon, and more preferred is dichloromethane. The organic solvent may be any mixture of the above-mentioned solvents in the appropriate ratio. In such a case, a mixture of halogenated hydrocarbon and alcohol is preferred, and a mixture of dichloromethane and ethanol is more preferred.

The concentration of the polymer in the organic solvent solution may vary depending on the molecular weight of the polymer or the type of the organic solvent. In a case where dichloromethane is used as the organic solvent, for example, such a concentration is selected from the range of generally from about 0.5 to about 70% by weight, more preferably from about 1 to about 60% by weight, particularly preferably from about 2 to about 50% by weight.

In a case where a mixture of dichloromethane and ethanol is used as the organic solvent, the content of ethanol in the mixture solvent is selected from the range of generally from about 0.01 to about 50% (v/v), more preferably from about 0.05 to about 40% (v/v), particularly preferably from about 0.1 to about 30% (v/v).

To the solution of the polymer in an organic solvent thus obtained, the physiologically active substance or a salt thereof is added and then dissolved or dispersed. In this process, the physiologically active substance or a salt thereof is added in such an amount that the weight ratio of the physiologically active substance or a salt thereof to the polymer is not more than about 1:1, preferably about 1:2.

The resulting solution of a composition comprising the physiologically active substance or a salt thereof and the polymer in an organic solvent is then added to an aqueous phase to form an O(oil phase)/W(aqueous phase)-type emulsion. Thereafter, the solvent is evaporated from the oil phase so that microspheres are prepared. In this process, the volume of the aqueous phase is selected from the range of generally from about 1 time to about 10,000 times, more preferably from about 5 times to about 50,000 times, particularly preferably from about 10 times to about 2,000 times the volume of the oil phase.

Besides the osmotic pressure regulating agent, an emulsifier may also be added to the outer aqueous phase. Such an emulsifier may be any emulsifier capable of forming a stable O/W-type emulsion. Specific examples of such an emulsifier include an anionic surfactant (such as sodium oleate, sodium stearate and sodium lauryl sulfate), a nonionic surfactant [such as polyoxyethylene sorbitan fatty acid ester (such as Tween 80 and Tween 60, Atlas Powder Company) and polyoxyethylene castor oil derivative (such as HCO-60 and HCO-50, Nikko Chemicals)], polyvinylpyrrolidone, polyvinyl alcohol, carboxymethyl cellulose, lecithin, gelatin, and hyaluronic acid. One or more of the above-mentioned emulsifiers may be used alone or in combination. It is preferably used in a concentration of about 0.01 to 10% by weight, more preferably of about 0.05 to about 5% by weight.

The organic solvent may be removed by a known method or a modified method based on the known method. Examples of such a method include a method comprising evaporating the organic solvent under normal atmospheric pressure or gradually reduced pressure while stirring with a propeller stirrer, a magnetic stirrer or the like, and a method comprising evaporating the organic solvent under a regulated vacuum with a rotary evaporator or the like.

The microspheres thus obtained are collected by centrifugation or filtration, washed with distilled water several times to remove the free physiologically active substance, the emulsifier and the like adhered to the surfaces of the microspheres, dispersed in distilled water or the like again, and then lyophilized.

During the process of producing the microspeheres, an antiflocculant may be added for preventing flocculation of the particles. Examples of such an antiflocculant include mannitol, lactose, glucose, water-soluble polysaccharide such as starch (such as cornstarch), amino acid such as glycine, and protein such as fibrin and collagen. In particular, mannitol is preferred.

The antiflocculant such as mannitol is generally added in an amount of 0 to about 24% by weight based on the total amount of the microspheres.

After the lyophilization, if desired, water and the organic solvent may be removed from the microspheres under reduced pressure by heating under such conditions that the microspheres are not fused with each other. Preferably, the microspheres are heated at about the intermediate glass transition temperature of the polymer, which is determined with a differential scanning calorimeter under the condition of the temperature rising rate of 10°C to 20°C per minute, or a slightly higher temperature than the intermediate glass transition temperature. More preferably, the microspheres are heated at about the intermediate glass transition temperature of the polymer to about 30°C higher temperature than the glass transition temperature. The heating time may vary depending on the amount of the microspheres or the like. It is generally from about 12 hours to about 168 hours, preferably from about 24 hours to about 120 hours, particularly preferably from about 48 hours to about 96 hours, after the microspheres reach the desired temperature.

A method for heating the microspheres may be any method capable of heating a population of microspheres uniformly and is not particularly limited.

Examples of the heat drying method include a method of heat drying in a constant-temperature bath, a fluidized-bed bath, a mobile bath or a kiln and a method of heat drying with a microwave. Preferred is a method of heat drying in a constant-temperature bath.

### (II) W/O/W Method

First, a solution of the polymer in an organic solvent is prepared.

Examples of the organic solvent include halogenated hydrocarbon (such as dichloromethane, chloroform, dichloroethane, trichloroethane, and carbon tetrachloride), ether (such as ethyl ether and isopropyl ether), fatty acid ester (such as ethyl acetate and butyl acetate), aromatic hydrocarbon (such as benzene, toluene and xylene), alcohol (such as ethanol and methanol), and acetonitrile. Preferred is halogenated hydrocarbon, and more preferred is dichloromethane. The organic solvent may be any mixture of the above-mentioned solvents in the appropriate ratio. In such a case, a mixture of halogenated hydrocarbon and alcohol is preferred, and a mixture of dichloromethane and ethanol is more preferred.

The concentration of the polymer in the organic solvent solution may vary depending on the molecular weight of the polymer or the type of the organic solvent. In a case where dichloromethane is used as the organic solvent, for example, such a concentration is selected from the range of generally from about 0.5 to about 70% by weight, more preferably from about 1 to about 60% by weight, particularly preferably from about 2 to about 50% by weight.

To the solution of the polymer in an organic solvent (an oil phase), the physiologically active substance, a salt thereof or a solution of the salt [wherein the solvent is water or a mixture of water and alcohol (such as methanol or ethanol)] is then added. The resulting mixture is emulsified by any known method such as with a homogenizer or sonication to form a W/O-type emulsion.

The resulting W/O-type emulsion comprising the physiologically active substance or a salt thereof and the polymer is then added to an aqueous phase to form a W(inner aqueous phase)/O(oil phase)/W(outer aqueous phase)-type emulsion. Thereafter, the solvent is evaporated from the oil phase so that microspheres are prepared. In this process, the volume of the outer aqueous phase is selected from the range of generally from about 1 time to about 10,000 times, more preferably from about 5 times to about 50,000 times, particularly preferably from about 10 times to about 2,000 times the volume of the oil phase.

The osmotic pressure regulating agent and emulsifier that may be optionally added to the outer aqueous phase and the subsequent preparation method may be the same as described in the above section (I).

An emulsifier may be added to the outer aqueous phase. Such an emulsifier may be any emulsifier capable of forming a stable O/W-type emulsion. Specific examples of such an emulsifier include an anionic surfactant (such as sodium oleate, sodium stearate and sodium lauryl sulfate), a nonionic surfactant [such as polyoxyethylene sorbitan fatty acid ester (such as Tween 80 and Tween 60, Atlas Powder Company) and polyoxyethylene castor oil derivative (such as HCO-60 and HCO-50, Nikko Chemicals)], polyvinylpyrrolidone, polyvinyl alcohol, carboxymethyl cellulose, lecithin, gelatin, and hyaluronic acid. One or more of the above-mentioned emulsifiers may be used alone or in combination. It is preferably used in a concentration of about 0.01 to 10% by weight, more preferably of about 0.05 to about 5% by weight.

The organic solvent may be removed by a known method or a modified method based on the known method. Examples of such a method include a method comprising evaporating the organic solvent under normal atmospheric pressure or gradually reduced pressure while stirring with a propeller stirrer, a magnetic stirrer, an ultrasonic generator or the like, a method comprising evaporating the organic solvent under a regulated vacuum with a rotary evaporator or the like, and a method comprising gradually removing the organic solvent using a dialysis membrane.

The microspheres thus obtained are collected by centrifugation or filtration, washed with distilled water several times to remove the free physiologically active substance or a salt thereof, the drug carrier, the emulsifier and the like adhered to the surfaces of the microspheres, dispersed in distilled water or the like again, and then lyophilized.

During the process of producing the microspeheres, an antiflocculant may be added for preventing flocculation of the particles. Examples of such an antiflocculant include mannitol, lactose, glucose, water-soluble polysaccharide such as starch (such as cornstarch), amino acid such as glycine, and protein such as fibrin and collagen. In particular, mannitol is preferred.

The antiflocculant such as mannitol is generally added in an amount of 0 to about 24% by weight based on the total amount of the microspheres.

After the lyophilization, if desired, water and the organic solvent may be removed from the microspheres under reduced pressure by heating under such conditions that the microspheres are not fused with each other. Preferably, the microspheres are heated at about the intermediate glass transition temperature of the polymer, which is determined with a differential scanning calorimeter under the condition of the temperature rising rate of 10°C to 20°C per minute, or a slightly higher temperature than the intermediate glass transition temperature. More preferably, the microspheres are heated at about the intermediate glass transition temperature of the polymer to about 30°C higher temperature than the glass transition temperature. The heating time may vary depending on the amount of the microspheres or the like. It is generally from about 12 hours to about 168 hours, preferably from about 24 hours to about 120 hours, particularly preferably from about 48 hours to about 96 hours, after the microspheres reach the desired temperature.

A method for heating the microspheres may be any method capable of heating a population of microspheres uniformly and is not particularly limited.

Examples of the heat drying method include a method of heat drying in a constant-temperature bath, a fluidized-bed bath, a mobile bath or a kiln and a method of heat drying with a microwave. Preferred is a method of heat drying in a constant-temperature bath.

The microsphere of the present invention produced by the method of the present invention refers to an injectable spherical fine particle which can be dispersed in a solution. For example, its shape and form can be determined by observation with a scanning electron microscope. The microsphere may be in the form of a microcapsule or a microparticle, and the microcapsule is preferred.

The weight content of the physiologically active substance or a salt thereof in the microsphere of the present invention may vary depending on the type of the physiologically active substance or a salt thereof, the desired pharmacological effect, the desired effect duration, and the like. For example, when the physiologically active substance or a salt thereof is a physiologically active peptide or a salt thereof, the content may be from about 0.001 to about 50% by weight, preferably from about 0.02 to about 40% by weight, more preferably from about 0.1 to about 30% by weight, still more preferably about 0.1 to about 24% by weight, most preferably from about 3 to about 24% by weight, based on the total weight of the microsphere. When the physiologically active substance or a salt thereof is a non-peptidic physiologically active substance or a salt thereof, the content may be from about 0.01 to about 80% by weight, preferably from about 0.1 to about 50% by weight.

The weight content of the polymer in the microsphere of the present invention may be from about 50 to about 100% by weight, preferably from about 70 to about 100% by weight, more preferably from about 85 to about 95% by weight, based on the total weight of the microsphere.

The weight content of the drug carrier in the microsphere of the present invention may be from about 0.01 to about 50% by weight, preferably from about 0.1 to about 30% by weight, more preferably from about 5 to about 15% by weight, based on the total weight of the microsphere.

The microsphere of the present invention has few small pores on the surface and has a good dispersibility in a suspension for injection.

Since the microsphere of the present invention has such a good dispersibility, a large amount of the microspheres can be suspended in a suspension for injection. Thus, a suspension for injection can eventually contain a large amount of the physiologically active substance or a salt thereof, even if the microsphere does not contain a drug carrier such as hydroxynaphthoic acid.

The microsphere of the present invention may be administered, as it is or after formulation into various dosage forms, as an injection or implant for muscle, subcutis, organ or the like, a transmucosal agent for nasal cavity, rectum, uterus or the like, or an oral agent (such as a capsule (such as a hard capsule and a soft capsule), a solid preparation such as a granule and a powder, and a liquid preparation such as a syrup, an emulsion and a suspension) or the like.

For example, the microspheres of the present invention may be mixed with a dispersion medium such as a dispersing agent (such as a surfactant such as Tween 80 and HCO-60; and polysaccharide such as sodium hyaluronic acid, carboxymethylcellulose and sodium alginate), a preservative (such as methyl paraben and propyl paraben), and an isotonic agent (such as sodium chloride, mannitol, sorbitol, glucose, and proline) to prepare an aqueous suspension, or mixed with a dispersion medium such as a vegetable oil such as sesame oil and corn oil to prepare an oily suspension, so that a practical sustained-release injection can be prepared.

The particle diameters of the microspheres of the present invention for use in the suspension injection should be in such a range that they have a satisfactory dispersibility and a satisfactory ability to pass through a needle. For example, the microspheres have an average particle diameter of about 0.1 to 300 µm, preferably of about 0.5 to 150 µm, more preferably from about 1 to 100 µm.

The microspheres of the present invention may be formulated into a sterile preparation by any method including, but not limited to, sterile conditions during all preparation steps, sterilization with gamma radiation and addition of an antiseptic.

For the above sustained-release microsphere injection, an excipient (such as mannitol, sorbitol, lactose, and glucose) may be added to the above components of the suspension, and the suspension may be re-dispersed and then freeze-dried or spray-dried to obtain a solid. At the time of administration, distilled water for injection or any appropriate dispersion medium may be added to the solid to prepare a more stable sustained-release injection.

In a case where an excipient such as mannitol is added to the sustained-release microsphere injection, the content of the excipient may be from about 0 to 50% by weight, preferably from about 1 to 20% by weight, based on the total amount of the injection.

In a case where the sustained-release microsphere injection is dispersed in distilled water for injection or any appropriate dispersion medium at the time of administration, the content of the microspheres may be from about 1 to 80% by weight, preferably from about 10 to 60% by weight, based on the total amount of the dispersion medium and the microspheres.

The microspheres of the present invention may be formulated into an oral preparation according to any known method. For example, the microspheres of the present invention are mixed with an excipient (such as lactose, white sugar and starch), a disintegrator (such as starch and calcium carbonate), a binder (such as starch, gum arabic, carboxymethyl cellulose, polyvinylpyrrolidone, and hydroxypropyl cellulose), a lubricant (such as talc, magnesium stearate and polyethylene glycol 6000)or the like, compression-molded, and then, if necessary, coated by any known method for the purpose of masking the taste or giving enteric or sustained-release property to obtain a oral preparation. Examples of such a coating agent include hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by Rohm Company, Germany, methacrylic acid-acrylic acid copolymer), and a pigment such as titanium oxide and colcothar.

The microspheres produced according to the method of the present invention may be formulated into a nasal preparation in the form of a solid, semi-solid or liquid by any known method. For example, the solid nasal preparation may be made of the microspheres by themselves or may be produced by adding and mixing an excipient (such as glucose, mannitol, starch, and microcrystalline cellulose), a thickener (such as natural gum, a cellulose derivative and an acrylic acid polymer) or the like to form a powdered composition. The liquid nasal preparation may be produced as an oily or aqueous suspension in a similar manner to the above injection. The semi-solid preparation is preferably produced as an aqueous or oily gel or an ointment form. These nasal preparations may contain a pH regulator (such as carbonic acid, phosphoric acid, citric acid, hydrochloric acid, and sodium hydroxide), an antiseptic (such as a para-hydroxybenzoate ester, chlorobutanol and benzalkonium chloride) or the like.

The microspheres of the present invention may be formulated into a suppository in the form of an oily or aqueous solid or semi-solid or a liquid according to any known method. An oily base used for the above suppository may be any oily base that does not allow the microsphere to dissolve. Examples of such an oily base include glyceride of higher fatty acid [such as cacao butter and Witepsol-series products (Dynamite Nobel)], medium fatty acid [such as Miglyol-series products (Dynamite Nobel)], and a vegetable oil (such as sesame oil, soybean oil and cottonseed oil). Examples of an aqueous base include polyethylene glycols and propylene glycol. Examples of aqueous gel base include natural gums, cellulose derivatives, vinyl polymers, and acrylic acid polymers.

The microspheres of the present invention are preferably used as an injection.

The content of the microspheres of the present invention in the sustained-release composition of the present invention is preferably, but not limited to, at least about 70% by weight.

The microsphere of the present invention is less toxic and thus may be used as a safe pharmaceutical or the like for a mammal (such as human, bovine, swine, dog, cat, mouse, rat, and rabbit).

The dose of the microspheres of the present invention or sustained-release composition thereof may vary depending on the type and content of the physiologically active substance or a salt thereof as the main drug, the dosage form, the duration of release of the physiologically active substance or a salt thereof, the target disease, the target animal, or the like, but may be set so as to provide an effective amount of the physiologically active substance or a salt thereof. In a case where the sustained-release composition is a six month preparation, for example, the dose of the physiologically active substance or a salt thereof as the main drug may be selected from the range of about 0.01 mg to 10 mg/kg, more preferably about 0.05 mg to 5 mg/kg of body weight for an adult.

The dose of the microspheres may be selected from the range of about 0.05 mg to 50 mg/kg, more preferably from about 0.1 mg to 30 mg/kg of body weight for an adult.

The frequency of administration may be once every several weeks, once a month, once every several months (such as three, four or six months), or the like and appropriately selected depending on the type and content of the physiologically active substance or a salt thereof as the main drug, the dosage form, the duration of release of the physiologically active substance or a salt thereof, the target disease, the target animal, or the like.

The microsphere of the present invention or a sustained-release composition thereof may be used as an agent for preventing or treating various diseases depending on the type of the physiologically active substance or a salt thereof contained therein. In a case where the physiologically active substance or a salt thereof is an LH-RH derivative, for example, the microsphere of the present invention or a sustained-release composition thereof may be used as an agent for preventing or treating a hormone-dependent disease, especially hormone-dependent cancer (such as prostatic cancer, uterus cancer, breast cancer, and pituitary tumor); a sex hormone-dependent disease such as prostatic hypertrophy, endometriosis, hysteromyoma, precocious puberty, dysmenorrheal, amenorrhea, premenstrual syndrome, and multilocular ovarian syndrome; or such a disease as Alzheimer's disease and immunodeficiency; or may be used as an agent for contraception (or for preventing or treating infertility, if a rebound effect is used after the drug holiday). The microsphere of the present invention or a sustained-release composition thereof may also be used as an agent for preventing or treating benign or malignant tumor that is not dependent on sex hormone but sensitive to LH-RH.

In order to produce the microspheres having improved dispersibility according to the present invention, the osmotic pressure regulating agent may be used in an outer aqueous phase when an emulsion containing the physiologically active substance or a salt thereof and the polymer is subjected to in-water drying.

The present invention is more specifically described by means of the following reference examples and examples, which are not intended to limit the scope of the present invention.

### EXAMPLES

In the following reference examples and examples, weight average molecular weight is determined in terms of polystyrene molecular weight by gel permeation chromatography (GPC) using monodisperse polystyrene as a reference material, and the content of each polymer is calculated from each weight average molecular weight. Each measurement is performed in a high performance GPC system (HLC-8120GPC manufactured by Tosoh Corporation) with SuperH4000x2 and SuperH2000 columns (each manufactured by Tosoh Corporation) and a mobile phase of tetrahydrofuran at a flow rate of 0.6 ml/min. The detection is based on differential refractive index.

### Reference Example 1: Synthesis of High Molecular Weight Lactic Acid Polymer

To 230 ml of dehydrated xylene were added 4.1 ml of a 1.0 mol/l diethylzinc hexane solution, 1.35 g of tert-butyl lactate and 230 g of DL-lactide and underwent polymerization reaction at 120 to 130°C for about two hours. After the reaction was completed, 120 ml of dichloromethane was poured into the reaction liquid, and 230 ml of trifluoroacetic acid was added to cause a deprotection reaction. After the reaction was completed, 300 ml of dichloromethane was added to the reaction liquid, which was then poured into 2800 ml of isopropyl ether so that the desired product was precipitated. Re-precipitation with dichloromethane/isopropyl ether was repeated so that a lactic acid polymer with a weight average molecular weight of about 40000 was obtained.

### Reference Example 2

The polymer obtained in Reference Example 1 was dissolved in 600 ml of dichloromethane. After the resulting solution was washed with water until it became neutral, 70 g of an aqueous 90% lactic acid solution was added and allowed to react at 40°C. When the weight average molecular weight of the polymer dissolved in the reaction liquid became about 20,000, the reaction liquid was cooled to room temperature, and 600 ml of dichloromethane was poured to stop the reaction. The reaction liquid was then washed with water until it became neutral. After the washing with water, the reaction liquid was concentrated to dryness to give a lactic acid polymer. In the resulting lactic acid polymer, the amount of the terminal carboxyl group was about 80 µmol per 1 g of the polymer and the content of a polymer with a weight average molecular weight of 5000 or less was 7.29% by weight.

### Reference Example 3 (1)

The polymer obtained in Reference Example 1 was dissolved in 600 ml of dichloromethane. After the resulting solution was washed with water until it became neutral, 70 g of an aqueous 90% lactic acid solution was added and allowed to react at 40°C. When the weight average molecular weight of the polymer dissolved in the reaction liquid became about 20,000, the reaction liquid was cooled to room temperature, and 600 ml of dichloromethane was poured to stop the reaction. After the reaction liquid was washed with water until it became neutral, the reaction liquid was added dropwise to 2800 ml of isopropyl ether so that the desired lactic acid polymer was precipitated. The precipitate collected by decantation was dissolved in 600 ml of dichloromethane. The resulting solution was concentrated to dryness to give 160 g of a lactic acid polymer. In the resulting lactic acid polymer, the amount of the terminal carboxyl group was about 70 µmol per 1 g of the polymer. Table 1 shows the weight average molecular weights of the high molecular weight lactic acid polymers used, the weight average molecular weights of the lactic acid polymers produced by the hydrolysis, and the weight average molecular weights and molecular weight distribution of the obtained target lactic acid polymer.

### Reference Examples 3 (2) to (6)

Lactic acid polymers according to the present invention were obtained in a similar manner to Reference Example 3 (1). Table 1 shows the weight average molecular weights of the high molecular weight lactic acid polymers used, the weight average molecular weights of the lactic acid polymers produced by the hydrolysis, and the weight average molecular weights and molecular weight distribution of the obtained target lactic acid polymers.

**[Table 1]**

| | | Reference Example 3 | | | | | |
|---|---|---|---|---|---|---|---|
| | | (1) | (2) | (3) | (4) | (5) | (6) |
| Mw of High Molecular Weight Lactic Acid Polymer | | 40500 | 43600 | 40400 | 43300 | 38600 | 55000 |
| Mw of Hydrolysis Product | | 22200 | 22200 | 22700 | 22200 | 18600 | 27200 |
| Mw of Obtained Lactic Acid Polymer | | 22900 | 22200 | 21900 | 22300 | 19400 | 28200 |
| Molecular Weight Distribution (%) | 1 - 1000 | 0.03 | 0.07 | 0.00 | 0.01 | 0.08 | 0.04 |
| | 1 - 3000 | 0.95 | 1.12 | 0.87 | 0.09 | 1.45 | 0.62 |
| | 1 - 5000 | 3.86 | 4.17 | 3.89 | 3.92 | 4.89 | 2.50 |

Table 1 indicates that in each lactic acid polymer produced by the method according to the present invention, the content of a polymer with a weight average molecular weight of 5000 or less is at most about 5% by weight; the content of a polymer with a weight average molecular weight of 3000 or less is at most about 1.5% by weight; and the content of a polymer with a weight average molecular weight of 1000 or less is at most about 0.1% by weight.

### Comparative Example 1

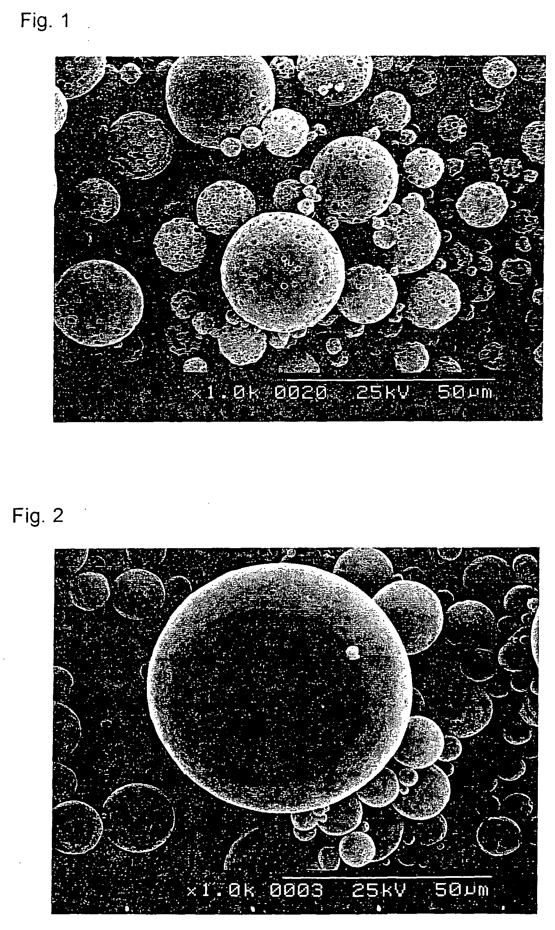

In 354.3 g of dichloromethane was dissolved 205.5 g of a DL-lactic acid polymer (with a weight average molecular weight of 21,400 and a carboxyl amount of 76.1 µmol/g determined by labeling quantitative determination) which was obtained in a similar manner to Reference Example 3 (1). The resulting solution was filtered under pressure with a 0.2 µm filter (DFA4201FRP, EMFLOW) and adjusted to 28.8°C. After 380.4 g of the resulting organic solvent solution was weighed out, it was mixed with an aqueous solution of 16.11 g of peptide A acetate in 16.22 g of distilled water, which was previously heated to 55.4°C. The mixture was stirred for 1 minute to be roughly emulsified and then emulsified at 10,150 rpm with a mini mixer for 2 minutes to form a W/O emulsion. After cooled to 18°C, the W/O emulsion was poured into 25 liters of an aqueous 0.1% (w/w) polyvinyl alcohol (EG-40 manufactured by The Nippon Synthetic) solution which was previously adjusted to 18.7°C over 3 minutes and 10 seconds and then stirred at 7,001 rpm with Homomic Line Flow (manufactured by Tokushu Kika Kogyo Co., Ltd.) to form a W/O/W emulsion. The temperature of the W/O/W emulsion was adjusted to about 18.5°C for 30 minutes and then stirred for 2 hours and 30 minutes without temperature adjustment so that dichloromethane and ethanol were volatilized or diffused into the outer aqueous phase and that the oil phase was solidified. After passed through a 75 µm mesh sieve, the microspheres were continuously precipitated and collected at 2,000 rpm with a centrifuge (H-600S manufactured by Kokusan Corporation). The collected microspheres were dispersed in a small amount of distilled water again and passed through a 90 µm mesh sieve. Thereto 18.85 g of mannitol was added and dissolved. The mixture was lyophilized to obtain microsphere powder. The mass and the yield of the resulting microsphere powder were 117.6 g and 68.54% respectively. The content of peptide A was 7.76%. An electron micrograph of the resulting microspheres is shown in Fig. 1.

### Example 1

In 354.4 g of dichloromethane was dissolved 205.4 g of a DL-lactic acid polymer (with a weight average molecular weight of 21,400 and a carboxyl amount of 76.1 µmol/g determined by labeling quantitative determination) which was obtained in a similar manner to Reference Example 3 (1). The temperature of the resulting solution was adjusted to 30°C. After 380.5 g of the resulting solution was weighed out, it was mixed with an aqueous solution of 16.1 g of leuprorelin acetate in 16.2 g of distilled water, which was previously heated at 55°C. The mixture was emulsified with a mini mixer (Tokushu Kika Kogyo Co., Ltd.) to form a W/O emulsion (at a rotation speed of about 10,000 rpm). After cooled to about 18°C, the W/O emulsion was poured into 25 liters of an aqueous 0.1% (w/w) polyvinyl alcohol (EG-40 manufactured by The Nippon Synthetic) + 1% mannitol solution which was previously adjusted to about 18°C, and then secondarily emulsified with Homomic Line Flow (manufactured by Tokushu Kika Kogyo Co., Ltd.) to form a W/O/W emulsion (at a turbine rotation speed of about 7,000 rpm and a circulating pump rotation speed of about 2000 rpm). The W/O/W emulsion was subjected to in-water drying for about 3 hours, passed through a standard 75 µm sieve, and then centrifuged (H-600S manufactured by Kokusan Corporation) to precipitate continuously and collect microspheres (at a rotation speed of about 2,000 rpm and a flow rate of about 600 ml/min). The collected microspheres were dispersed in a small amount of distilled water again and passed through a standard 90 µm sieve. Thereto 18.9 g of mannitol was added. The mixture was lyophilized with a lyophilizer (Triomaster manufactured by Kyowa Vacuum Engineering) to obtain powder (microsphere powder). An electron micrograph of the resulting microspheres is shown in Fig. 2.

### Experimental Example 1

About 660 mg of the microsphere powder produced in Comparative Example 1 or Example 1 was weighed in a coat 9P vial, which was then plugged with a rubber stopper and sealed with a screw cap. To the vial was added 1.5 ml of a dispersion medium for leuprorelin acetate (a mixture of 5% mannitol, 1% carmellose sodium and 0.1% polysorbate 80), and the time required for uniform dispersion to be attained was measured.

Each microsphere powder was dispersed by shaking at a shaking width of about 7 cm and a shaking speed of about 30 times/10 seconds according to instructions attached to a leuprorelin acetate vial preparation. The results are shown in Table 2.

**[Table 2]**

| | Comparative Example 1 | Example 1 |
|---|---|---|
| Dispersion Time | about 2 to 4 minutes | 8 to 23 seconds |

### Experimental Example 2

About 660 mg of the microsphere C powder produced in Comparative Example 1 or Example 1 was charged into a 14φ type DPS (dual-chamber prefilled syringe) which was filled with a dispersion medium for leuprorelin acetate (the amount of the dispersion liquid: 1.5 ml), and suspended. The time required for uniform dispersion to be attained was measured.

Each microsphere powder was dispersed by tapping the syringe on a palm at a shaking width of about 3 cm and at a shaking speed of about 50 times/10 seconds according to instructions attached to a leuprorelin acetate DPS preparation. The results are shown in Table 3.

**[Table 3]**

| | Comparative Example 1 | Example 1 |
|---|---|---|
| Dispersion Time | about 2 to 6 minutes | 20 to 46 seconds |

### Industrial Applicability

The microspheres of the present invention have improved dispersibility and thus can be dispersed at a high concentration in a dispersion medium such as distilled water for injection.

## Claims

1. Use of mannitol for improving dispersibility of microspheres, in which the mannitol is added in an outer aqueous phase in producing microspheres by an in-water drying method using a W/O type emulsion, wherein the W/O type emulsion consists of:
1) an inner aqueous phase containing a peptide represented by the formula: 5-oxo-Pro-His-Trp-Ser-Tyr-Y-Leu-Arg-Pro-Z wherein Y represents DLeu, DAla, DTrp, DSer(tBu), D2Na1 or DHis(ImBzl) and Z represents NH-C₂H₅ or Gly-NH₂, or a salt thereof and
2) an oil phase of a solution containing a lactic acid polymer with a weight average molecular weight of 15000 to 50000, wherein the lactic acid polymer consists of only lactic acid.

## Patentansprüche

1. Verwendung von Mannitol zur Verbesserung der Dispergierbarkeit von Mikrosphären, in welchen das Mannitol bei der Produktion von Mikrosphären durch ein In-Wasser-Trocknungsverfahren unter Verwendung einer Emulsion vom Typ W/O in eine äußere wässrige Phase hinzugegeben wird, wobei die Emulsion vom Typ W/O aus Folgendem besteht:
1) einer inneren wässrigen Phase, die ein Peptid enthält, das durch die folgende Formel dargestellt wird: 5-Oxo-Pro-His-Trp-Ser-Tyr-Y-Leu-Arg-Pro-Z, wobei Y DLeu, DAla, DTrp, DSer(tBu), D2Nal oder DHis(ImBzl) darstellt und Z NH-C₂H₅ oder Gly-NH₂ darstellt oder ein Salz davon und
2) eine Ölphase einer Lösung, die ein Milchsäurepolymer mit einem Gewichtsmittel des Molekulargewichts von 15000 bis 50000 enthält, wobei das Milchsäurepolymer nur aus Milchsäure besteht.

## Revendications

1. Utilisation du mannitol pour améliorer la dispersibilité de microsphères, dans laquelle le mannitol est ajouté à une phase aqueuse externe en produisant des microsphères par un procédé de séchage dans l'eau au moyen d'une émulsion du type E/H, ladite émulsion de type E/H se composant de :
1) une solution aqueuse interne contenant un peptide représenté par la formule : 5-oxo-Pro-His-Trp-Ser-Tyr-Y-Leu-Arg-Pro-Z dans laquelle Y représente DLeu, DAla, DTrp, DSer(tBu), D2Na1 ou DHis(ImBzl) et Z représente NH-C₂H₅ ou Gly-NH₂, ou un sel de ceux-ci et
2) une phase huileuse d'une solution contenant un polymère d'acide lactique ayant un poids moléculaire moyen en poids de 15000 à 50000, ledit polymère d'acide lactique se composant uniquement d'acide lactique.
